# EUROPEAN PATENT SPECIFICATION

(11) **EP 2 450 016 B1**
(45) Date of publication and mention of the grant of the patent: **27.05.2015**
(21) Application number: 11187451.7
(22) Date of filing: 02.11.2011
(51) Int. Cl.: A61F 13/505

(54) **Quick-attach sanitary absorbent article assembly**
Aufbau eines Sanitäraufnahmeartikels mit Schnellbefestigung
Ensemble d'article absorbant sanitaire à fixation rapide

(30) Priority: 03.11.2010 US 409790 P; 27.09.2011 US 201113246054
(43) Date of publication of application: 09.05.2012
(73) Proprietor: McNeil-PPC, Inc., Skillman, NJ 08558 (US)
(72) Inventor: Austin, Jennifer J., Trenton, NJ New Jersey 08611 (US); Dabi, Shmuel, Highland Park, NJ New Jersey 08903 (US); Poccia, John F., Monmouth Beach, NJ New Jersey 07750 (US)
(74) Representative: Carpmaels & Ransford LLP

(56) References cited:
- WO-A1-2010/101500

## Description

### FIELD OF THE INVENTION

The present invention relates to a quick-attach absorbent article assembly including a base assembly for attachment to an undergarment and an absorbent article structured to enable a user to quickly attach the absorbent article to the base assembly and easily remove the article from the base assembly when the article is soiled.

### BACKGROUND OF THE INVENTION

Disposable sanitary absorbent articles, such as sanitary napkins, panty liners, incontinence articles, and the like, are well known to those of skill in the art. Typically, such articles include a liquid-permeable body facing cover layer, a liquid-impermeable garment facing barrier layer, and an absorbent core arranged between the cover layer and the barrier layer. The garment facing surface of the barrier layer is typically provided with garment attachment adhesive that enables a user to securely attach the absorbent article to an undergarment during use. Prior to use, the garment attachment adhesive is usually covered with a removable release paper that protects the garment attachment adhesive from contamination prior to use. When ready for use, a user removes the release paper to thereby expose the garment attachment adhesive and places the article in the crotch portion of the user's undergarment so that the garment attachment adhesive secures the article to the crotch portion of the undergarment. Such absorbent articles may also be provided with wings that extend outwardly from a main body of the article. The garment facing surface of each wing is provided with a garment attachment adhesive and the wing is adapted to be folded around an edge of the undergarment and secured by way of the adhesive to an external surface of the undergarment, to thereby hold the article in place.

A problem with absorbent articles having the structure described above is that they can be difficult to apply to the undergarment and remove therefrom. For example, upon removal of the release paper the article may fold upon itself causing two different areas of the article to adhere to one another. Likewise, the wings of such article may fold towards the main body portion and adhere thereto prior to the user correctly positioning the article in the undergarment. Further, it is difficult to handle multiple release papers, especially for those articles including wings. In addition, once the article has been soiled it is difficult to remove the article in a sanitary manner.

In view of the above there is a need for an absorbent article assembly that allows an absorbent article to be easily attached to, and removed from, an undergarment.

### SUMMARY OF THE INVENTION

In view of the foregoing, the present invention provides a quick-attach sanitary absorbent article assembly including a base assembly selectively attachable to an undergarment, and an absorbent article,
wherein said base assembly consists of (i) a base substrate having a garment facing surface provided with a garment attachment adhesive and an opposed upper surface and (ii) a fastener substrate that is arranged on top of, and secured to, the upper surface of the base substrate, wherein said fastener substrate includes a plurality of protrusions,
wherein said base substrate is formed from liquid-impermeable materials or is rendered liquid-impermeable by a separate treatment, and
wherein the absorbent article is structured and arranged to be engaged by and held in place in the undergarment by the plurality of protrusions.

### BRIEF DESCRIPTION OF THE DRAWINGS

Fig. 1 is a perspective view showing how the absorbent article assembly according to the present invention is installed into a crotch portion of an undergarment;
Fig. 2 is a perspective view similar to Fig. 1 from an underside of the undergarment;
Fig. 3 is a partially exploded view of the base assembly that forms part of the absorbent article assembly according to the present invention;
Fig. 4 is a sectional view taken along line 4-4 in Fig. 3;
Fig. 5 is an exploded view of the absorbent article that forms part of the absorbent article assembly shown in Fig. 1;
Fig. 6 is a sectional view taken along line 6-6 in Fig. 5; and
Fig. 7 is a perspective view showing how a soiled absorbent article is removed from the base assembly and replaced with a fresh absorbent article.

### DETAILED DESCRIPTION OF THE INVENTION

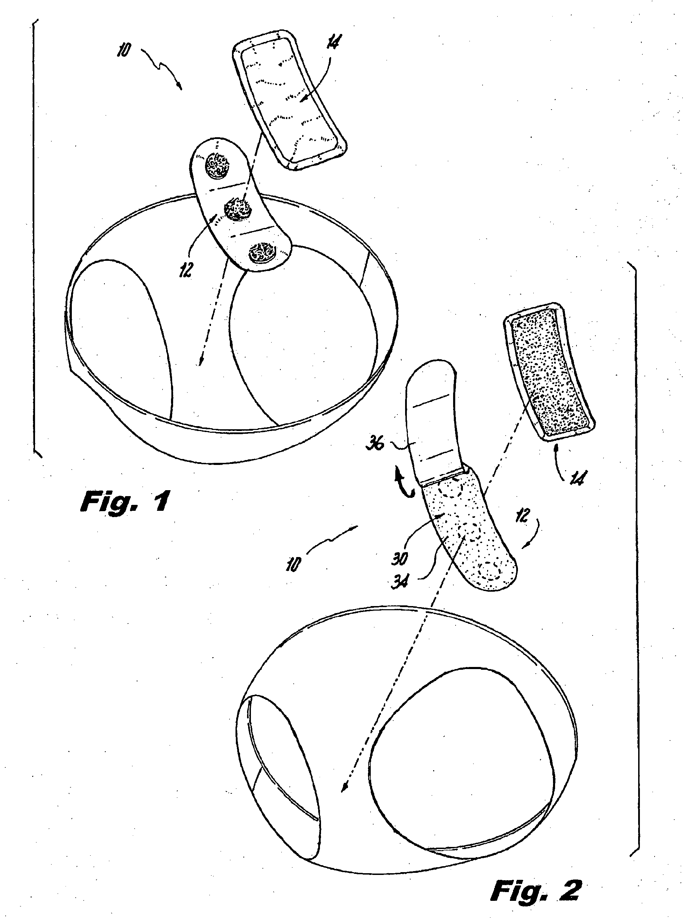

The present invention will be described herein with reference to a sanitary napkin, however the present invention is equally applicable to other sanitary absorbent articles such as panty liners, adult incontinence products and the like. Referring to Figs. 1 and 2, the quick-attach absorbent article assembly **10** according to the present invention generally includes a base assembly **12** and a sanitary napkin **14.**

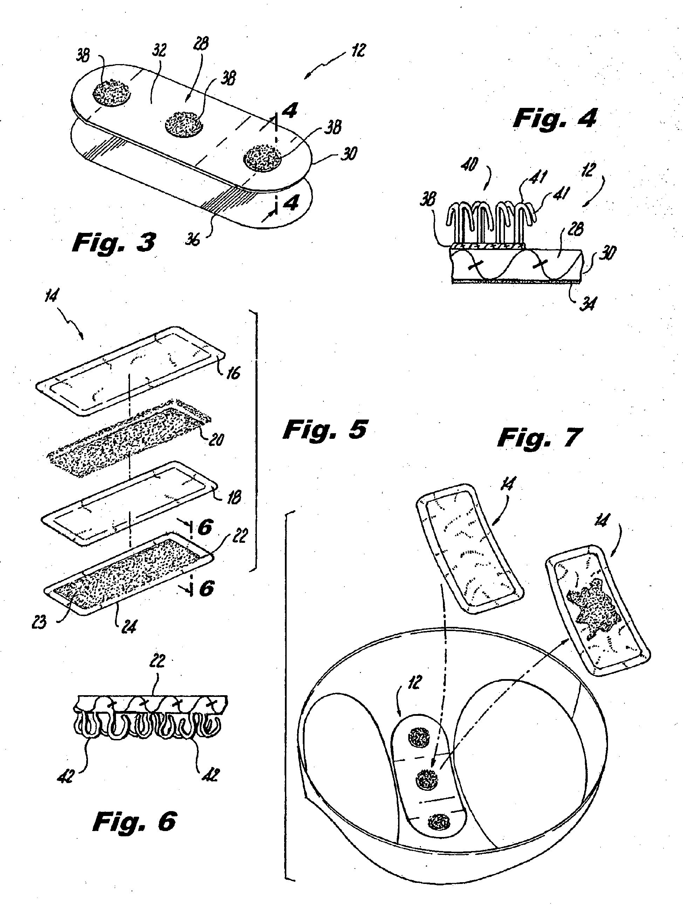

Referring to Fig. 5, the sanitary napkin **14** includes a liquid- permeable body facing cover layer **16,** an attachment layer **22,** and an absorbent core **20** arranged between the cover layer **16** and the attachment layer **22.** The sanitary napkin **14** may optionally include a liquid-impermeable barrier layer 18 arranged between the absorbent core **20** and the attachment layer 22.

The attachment layer **22** includes an absorbent core facing surface **23** and a base assembly facing surface **24.** As will be described in greater detail below, the attachment layer **22** is structured and arranged to engage, and be retained by, the base assembly **12.** The sanitary napkin **14** may optionally further include a transfer layer arranged between the cover layer **16** and the absorbent core **20** (not shown in the Figures).

Referring to Figs. 3 and 4, the base assembly **12** generally includes a base substrate **28** having a garment facing surface **30** and an opposed upper surface **32.** As best seen in Figs. 2 and 4, the garment facing surface **30** is provided with a garment attachment adhesive **34.** The garment attachment adhesive **34** is adapted to enable a user to selectively attach the base assembly **12** to an undergarment during use. Prior to use, the garment attachment adhesive **34** may be covered with a removable release paper **36** to protect the adhesive **34** as shown in Figs. 2 and 3.

The base assembly **12** further includes a fastener substrate **38** that is arranged on top of, and secured to, the upper surface **32** of the base substrate **28.** The fastener substrate **38** may be secured to the base substrate **28** by any conventional commercially available construction adhesive, or by any other conventional known means such as heat sealing or the like. As shown in Fig. 4, the fastener substrate **38** includes a plurality of upwardly extending protrusions **40** that are adapted to engage the attachment layer **22** of the sanitary napkin **14** and thereby retain the sanitary napkin **14** securely attached to the base assembly **12** during use. As shown in Fig. 4, each protrusion may comprise a hook structure **41.** The attachment layer **22** of the sanitary napkin **14** is preferably formed from a woven or nonwoven material. As shown in Fig. 6, the attachment layer **22,** whether woven or nonwoven, should define a plurality of loops **42** that extend towards the base assembly **12** and are adapted to engage with and be held by the hook structures **41** of the base assembly **12.**

As shown in Fig. 3, the fastener substrate **38** may be arranged in a plurality of distinct islands, i.e. each fastener substrate **38** may be arranged in spaced relationship to an adjacent fastener substrate **38.** Alternatively, the fastener substrate **38** could be arranged as a continuous layer that extends in an uninterrupted manner.

As described below, the above described structure allows a user to easily attach a sanitary napkin **14** to the base assembly **12,** and when the sanitary napkin is soiled, easily remove the soiled sanitary napkin 14 and replace the same with a fresh sanitary napkin **14.**

A description of the manner in which the quick-attach absorbent article assembly **10** is used will now be provided. When a user is ready to use the assembly **10** the user first removes the removable release paper **36** from the base assembly **12** to thereby expose the garment attachment adhesive **34**, as shown in Fig. 2. Thereafter, the user manually places the base assembly **12** in the crotch portion of the user's undergarment so that the garment facing surface **30** of the base assembly **12** is facing the user's undergarment. By pressing the base assembly **12** against the crotch portion of the undergarment the user may securely attach the base assembly **12** thereto by means of the garment attachment adhesive **34**. Once the base assembly **12** is in place the user then arranges the absorbent article **14** on top of the base assembly **12** such that the base assembly facing surface **24** of the attachment layer **22** faces the base assembly **12**. By pressing firmly down on the article **14** the protrusions **40** of the base assembly **12** engage the attachment layer **22** and thereby function to hold the article **14** in place for use. Once the article **14** has been soiled the user may manually remove the article **14** and replace the same with a new fresh article **14**. During this replacement process the base assembly **12** stays in place in the crotch portion of the undergarment thereby significantly simplifying the change experience of the user. It is important that the garment attachment adhesive **34** be selected so that the effective removal force required to remove the base assembly **12** from the undergarment is greater than the removal force required to remove the sanitary napkin **14** from the base assembly **12**. This will insure that the base assembly **12** will stay in place in the undergarment when the article **14** is removed from the base assembly **12**. The base assembly **12** is removable from the undergarment prior to laundering.

### Cover Layer

The liquid-permeable body facing cover layer **16** may be a relatively low density, bulky, high-loft non-woven web material. The cover layer **16** may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof.

The cover layer **16** preferably has a relatively high degree of wettability, although the individual fibers comprising the cover may not be particularly hydrophilic. The cover material should also contain a great number of relatively large pores. This is because the cover layer **16** is intended to take-up body fluid rapidly then transport it away from the body and the point of deposition. Therefore, the cover layer contributes little to the time taken for the napkin to absorb a given quantity of liquid (penetration time).

Advantageously, the fibers which make up the cover layer **16** should not lose their physical properties when they are wetted, in other words they should not collapse or lose their resiliency when subjected to water or body fluid. The cover layer **16** may be treated to allow fluid to pass through it readily. The cover layer **16** also functions to transfer the fluid quickly to the underlying layers of the napkin. Thus, the cover layer **16** is advantageously wettable, hydrophilic and porous. When composed of synthetic hydrophobic fibers such as polyester or bi-component fibers, the cover layer **16** may be treated with a surfactant to impart the desired degree of wettability.

Alternatively, the cover layer **16** can also be made of polymer film having large pores. Because of such high porosity, the film accomplishes the function of quickly transferring body fluid to the inner layers of the underlying absorbent layers.

The cover layer **16** may be attached to the underlying layers of the sanitary napkin **14** by adhesion and/or other suitable means know to those of skill in the art.

The cover layer may comprise a hot through air bonded nonwoven composed of PE/PET bicomponent fibers, such nonwoven being commercially available from Shalag Industries, Ltd., Upper Galilee, Israel.

### Transfer Layer

The optional transfer layer (not shown in the Figures) may be composed of fibrous materials, such as wood pulp, polyester, rayon, flexible foam, or the like, or combinations thereof. The transfer layer may also optionally include a superabsorbent polymer (SAP) material. The transfer layer may also comprise thermoplastic fibers for the purpose of stabilizing the layer and maintaining its structural integrity. The transfer layer may be treated with surfactant on one or both sides in order to increase its wettability, although generally the transfer layer is relatively hydrophilic and may not require treatment. The transfer layer is preferably bonded on both sides to the adjacent layers, i.e. to the cover layer 16 and the underlying core 20.

The transfer layer may comprise an airlaid pulp material commercially available from P.H. Glatfelter Company, York, PA, under product code MH090.102.

### Absorbent Core

The absorbent core **20** may comprise a single layer of material or may comprise multiple layers. The core **20** may be a blend or mixture of cellulosic fibers and superabsorbent disposed therein. Cellulosic fibers that can be used in the second absorbent layer **20** are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, crosslinking agents and the like for use in the present material. Some portion of the pulp may be chemically treated as discussed in U.S. Pat. No. 5,916,670 to improve flexibility of the product. Flexibility of the material may also be improved by mechanically working the material or tenderizing the material.

The core **20** can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.

In a specific example, the core **20** is a material containing from 90% to about 40% percent cellulosic fiber, about 10% to about 60% SAP. The core **20** may comprise a material manufactured by using air-laying means well known in the art.

The core **20** may be relatively thin, high swelling absorbent material sold under the trade name NOVATHIN ® INT-002 available from EAM Corporation located in Jessup, Ga., U.S.A.

The absorbent core **20** may be adhered to the adjacent layers, e.g. the cover layer **16** (or transfer layer if such layer is employed) and the barrier layer **18** by means of a conventional construction adhesive well known to those of skill in the art.

### Barrier Layer

Underlying absorbent core is the liquid-impermeable barrier layer **18.** The barrier layer **18** preferably comprises liquid-impervious film material so as to prevent liquid that is entrapped in the absorbent core **20** from egressing the sanitary napkin **14** and staining the wearer's undergarment. The barrier layer **18** is preferably made of polymeric film, although it may be made of liquid-impervious, air-permeable material such as repellent-treated non-woven or micropore films or foams.

The barrier layer **18** may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer **16** and the barrier layer **18** are preferably joined along their marginal portions so as to form an enclosure or flange seal that maintains the absorbent core **20** captive. The joint may be made by means of adhesives, heat-bonding, ultrasonic bonding, radio frequency sealing, mechanical crimping, and the like and combinations thereof.

The barrier layer **18** may comprise a polyethylene film material of the type commercially available from Berry Plastics Corporation, Evansville, IN.

### Attachment Layer

The attachment layer **22** is preferably formed from a woven or nonwoven material. Nonwoven materials may include spunmelts, thermo-bonds, through air bonds, needlepunch or spunlace materials. Alternatively the attachment layer may be formed from polymeric films provided that such films are provided with a sufficient structure to engage with and be retained by the protrusions **40** of the base assembly **12.** For example such films may be provided with holes, fibrillated, or provided with some other three dimensional structure that enables the film to engage with and be retained by the protrusions **40.**

As shown in Fig. 6, the attachment layer **22** may define a plurality of loops **42** that extend towards the base assembly **12** and are adapted to engage with and be held by the hook structures **41** of the base assembly **12.** In this manner, the loops **42** and hook structures **41** function to hold the sanitary napkin **14** in place during use and also permit the user to manually remove the napkin **14** once the napkin **14** has been soiled. The attachment layer **22** may comprise a single layer or may comprise a multi-layer laminate provided that such laminate includes a layer that faces the base assembly having a plurality of loops **42** as described herein. The attachment layer **22** is preferably secured to the barrier layer **18,** or core structure if such barrier **18** is omitted, by means of a conventional construction adhesive well known to those of skill in the art.

The attachment layer **22** may comprise a liquid-impermeable, air permeable, laminate structure including a microporous film core facing layer that is bonded to a spunbond nonwoven base assembly facing layer. Since the attachment layer **22** is liquid-impermeable the barrier **18** may be omitted if desiredThe attachment layer **22** may comprise a 35 gsm laminate structure of this type commercially available from Kang Na Hsiung Enterprises Co., Ltd., Taipei, Taiwan under product code CL035-CPSS=N0.

### Base Substrate

The base substrate **28** may be formed from any conformable material such as a suitably flexible woven or nonwoven material that will move with the user's undergarment. The base substrate **28** may be formed of a single layer or may be formed from a multi-layer laminate. The base substrate **28** is formed from liquid-impermeable materials or is rendered liquid-impermeable by a separate treatment. As discussed above, the garment facing surface **30** of the base substrate **28** is provided with a garment attachment adhesive **34.**

The garment attachment adhesive **34** may be any suitable pressure-sensitive adhesive. As used herein, the term pressure-sensitive adhesive refers to any releasable adhesive or releasable tenacious means. Suitable adhesive compositions, include, for example, water-basted pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on acrylics or suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof; hot melt adhesives based upon acrylic polymers. Commercial examples of these types of block copolymers include Kraton™ elastomers from Shell Chemical Company, Vector™ elastomers from Dexco, Solprene™ from Enichem Elastomers and Stereon™ from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva™ (polymers from AT plastics), Nucrel™ ( polymers from DuPont), Escor™ (from Exxon Chemical). The garment attachment adhesive may be an acrylic hot melt Duro-Tak 34-546B from Henkel AG & Co. KGaA, Dusseldorf, Germany.

The base substrate **28** may comprise a liquid- impermeable, air permeable, laminate structure including a microporous film garment facing layer that is bonded to a spunbond nonwoven fastener substrate facing layer. Laminate structures of this type are commonly used as a backsheet material for diapers. The attachment layer **22** may comprise a 35 gsm laminate structure of this type commercially available from Kang Na Hsiung Enterprises Co., Ltd., Taipei, Taiwan under product code CL035-CPSS-N0.

### Fastener Substrate

The fastener substrate **38** is preferably formed from a polymeric material including a plurality of upwardly extending protrusions **40** as described above. Each protrusion may comprise a hook structure **41.** Polymeric materials of this type are commercially available from Velcro USA, Inc., Manchester, NH.

The fastener substrate **38** may comprise a polymeric material commercially available from Velcro USA, Inc., Manchester, NH under product code HTH 847.

The fastener substrate **38** is bonded to the article facing surface of the base substrate **28** by any conventional known means in the art such as adhesion or ultrasonic bonding.

### EXAMPLE

A quick-attach absorbent article assembly according to one embodiment of the invention was constructed as follows:

### Sanitary Napkin

Cover:
   HTA 27 gsm bico fibers PE/PET, STA4ETW27, Shalag Industries, Ltd, Upper Galilee, Israel.
Adhesive layer:
   NW 1023 1.0 gsm (100% coverage) stryrenated block copolymer based hot melt pressure sensitive adhesive, HB Fuller, St Paul, MN.
Transfer layer:
   90 gsm airlaid 90% pulp, MH090.102, P.H. Glatfelter Company, York, PA.
Adhesive layer:
   NW 1023 4.0 gsm (100% coverage,) stryrenated block copolymer based hot melt pressure sensitive adhesive, HB Fuller, St Paul, MN.
Core:
   Novathin 208 gsm 25% SAP, 56mm x 205 mm, Novathin INT-002, EAM corporation, Jesup, GA.
Adhesive layer:
   NW 1023 4.0 gsm (100% coverage,) stryrenated block copolymer based hot melt pressure sensitive adhesive, HB Fuller, St Paul, MN.
Barrier:
   Pliant, 0.8 mil PE metallocene, Berry Plastics Corporation, Evansville, IN.
Adhesive layer:
   NW 1042 22.0 gsm (∼40% coverage,) stryrenated block copolymer based hot melt pressure sensitive adhesive, HB Fuller, St Paul, MN
Attachment layer:
   a 35 gsm laminate structure of this type commercially available from Kang Na Hsiung Enterprises Co., Ltd., Taipei, Taiwan under product code CL035-CPS5-N0.

### Base Assembly

Base substrate:
   a 35 gsm laminate structure of this type commercially available from Kang Na Hsiung Enterprises Co., Ltd., Taipei, Taiwan under product code CL035-CPS5-N0.
Garment attachment adhesive on a garment facing surface of the base substrate:
   an acrylic hot melt Duro-Tak 34-546B from Henkel AG & Co. KGaA, Dusseldorf, Germany.
Fastener substrate:
   a polymeric material commercially available from Velcro USA, Inc., Manchester, NH under product code HTH 847.
Adhesive between base substrate and fastener substrate:
   NW 1042 22.0 gsm stryrenated block copolymer based hot melt pressure sensitive adhesive, HB Fuller, St Paul, MN

The application therefore discloses, *inter alia:*
(i) A quick-attach absorbent article assembly comprising:
   a base assembly selectively attachable to an undergarment, said base assembly including a plurality of protrusions; and
   an absorbent article;
   wherein said absorbent article is structured and arranged to be engaged by and held in place in said undergarment by said protrusions.
(ii) The quick-attach absorbent article assembly according to (i), wherein each of said protrusions comprises a hook structure.
(iii) The quick-attach absorbent article assembly according to (ii), wherein said base assembly comprises a base substrate and a fastener substrate.
(iv) The quick-attach absorbent assembly according to (iii), wherein said fastener substrate includes a plurality of said hook structures.
(v) The quick-attach absorbent article assembly according to (iv), wherein a garment facing surface of said base substrate includes a garment attachment adhesive adapted to allow a user to selectively attach the base assembly to said undergarment.
(vi) The quick-attach absorbent article assembly according to (v), wherein said absorbent article comprises an attachment layer.
(vii) The quick-attach absorbent article assembly according to (vi), wherein said attachment layer includes a plurality of loops, said loops being structured and arranged to be engaged and retained by said hook structures.
(viii) The quick-attach absorbent article assembly according to (vii), wherein said absorbent article further comprises a liquid-permeable body facing cover layer.
(ix) The quick-attach absorbent article assembly according to (viii), wherein said absorbent article further comprises an absorbent core arranged between said cover layer and said attachment layer.

## Claims

1. A quick-attach absorbent article assembly comprising:
a base assembly selectively attachable to an undergarment; and
an absorbent article;
wherein said base assembly consists of (i) a base substrate having a garment facing surface provided with a garment attachment adhesive and an opposed upper surface and (ii) a fastener substrate that is arranged on top of, and secured to, the upper surface of the base substrate, wherein said fastener substrate includes a plurality of protrusions,
wherein said base substrate is formed from liquid-impermeable materials or is rendered liquid-impermeable by a separate treatment, and
wherein said absorbent article is structured and arranged to be engaged by and held in place in said undergarment by said protrusions.

2. The quick-attach absorbent article assembly according to claim 1, wherein each of said protrusions comprises a hook structure.

3. The quick-attach absorbent article assembly according to any preceding claim, wherein a garment facing surface of said base substrate includes a garment attachment adhesive adapted to allow a user to selectively attach the base assembly to said undergarment.

4. The quick-attach absorbent article assembly according to claim 3, wherein the garment attachment adhesive is covered with a removable release paper.

5. The quick-attach absorbent article assembly according to any preceding claim, wherein said absorbent article comprises an attachment layer.

6. The quick-attach absorbent article assembly according to claim 5, wherein said attachment layer includes a plurality of loops, said loops being structured and arranged to be engaged and retained by said hook structures.

7. The quick-attach absorbent article assembly according to any preceding claim, wherein said absorbent article comprises a liquid-permeable body facing cover layer.

8. The quick-attach absorbent article assembly according to any preceding claim, wherein said absorbent article comprises an absorbent core arranged between said cover layer and said attachment layer.

## Patentansprüche

1. Aufbau eines saugfähigen Artikels mit Schnellbefestigung, die Folgendes aufweist:
einen Grundaufbau, der sich selektiv an Unterwäsche befestigen lässt; und
einen saugfähigen Artikel;
wobei der Grundaufbau besteht aus: (i) einem Grundsubstrat, das eine zur Bekleidung weisende Oberfläche aufweist, die mit einem Bekleidungsbefestigungsklebemittel versehen ist, und eine gegenüberliegende Oberseite und (ii) ein Befestigungssubstrat, das auf der Oberseite des Grundsubstrats angeordnet und daran gesichert ist, wobei das Befestigungssubstrat mehrere Vorsprünge aufweist,
wobei das Grundsubstrat aus flüssigkeitsundurchlässigen Materialien ausgebildet ist oder durch eine getrennte Behandlung flüssigkeitsundurchlässig gemacht ist, und
wobei der saugfähige Artikel so strukturiert und eingerichtet ist, dass er durch die Vorsprünge in Eingriff genommen und in der Unterwäsche an Ort und Stelle gehalten wird.

2. Aufbau eines saugfähigen Artikels mit Schnellbefestigung nach Anspruch 1, wobei jeder der Vorsprünge eine Hakenstruktur aufweist.

3. Aufbau eines saugfähigen Artikels mit Schnellbefestigung nach einem der vorhergehenden Ansprüche, wobei eine zur Bekleidung weisende Oberfläche des Grundsubstrats ein Bekleidungsbefestigungsklebemittel umfasst, das eingerichtet ist, es einem Benutzer zu ermöglichen, den Grundaufbau selektiv an der Unterwäsche zu befestigen.

4. Aufbau eines saugfähigen Artikels mit Schnellbefestigung nach Anspruch 3, wobei das Bekleidungsbefestigungsklebemittel mit einem entfernbaren Trennpapier abgedeckt ist.

5. Aufbau eines saugfähigen Artikels mit Schnellbefestigung nach einem der vorhergehenden Ansprüche, wobei der saugfähige Artikel eine Befestigungsschicht aufweist.

6. Aufbau eines saugfähigen Artikels mit Schnellbefestigung nach Anspruch 5, wobei die Befestigungsschicht mehrere Schlingen aufweist, wobei die Schlingen strukturiert und eingerichtet sind, durch die Hakenstruktur in Eingriff genommen und durch sie gehalten zu werden.

7. Aufbau eines saugfähigen Artikels mit Schnellbefestigung nach einem der vorhergehenden Ansprüche, wobei der saugfähige Artikel eine flüssigkeitsdurchlässige, zum Körper weisende Deckschicht aufweist.

8. Aufbau eines saugfähigen Artikels mit Schnellbefestigung nach einem der vorhergehenden Ansprüche, wobei der saugfähige Artikel einen saugfähigen Kern aufweist, der zwischen der Deckschicht und der Befestigungsschicht angeordnet ist.

## Revendications

1. Ensemble d'article absorbant à fixation rapide, comprenant:
un ensemble de base qui peut être attaché de façon sélective à un sous-vêtement; et
un article absorbant,
dans lequel ledit ensemble de base est constitué (i) d'un substrat de base présentant une surface opposée au vêtement qui est pourvue d'un adhésif de fixation au vêtement et d'une surface supérieure opposée, et (ii) d'un substrat de fixation qui est agencé au-dessus de et est fixé à la surface supérieure du substrat de base, dans lequel ledit substrat de fixation comporte une pluralité de saillies,
dans lequel ledit substrat de base est constitué de matériaux imperméables au liquide ou est rendu imperméable au liquide par un traitement séparé, et
dans lequel ledit article absorbant est structuré et agencé de manière à être engagé par et maintenu en place dans ledit sous-vêtement par lesdites saillies.

2. Ensemble d'article absorbant à fixation rapide selon la revendication 1, dans lequel chacune desdites saillies comprend une structure à crochets.

3. Ensemble d'article absorbant à fixation rapide selon l'une quelconque des revendications précédentes, dans lequel une surface opposée au vêtement dudit substrat de base comprend un adhésif de fixation au vêtement qui est adapté pour permettre à un utilisateur d'attacher de façon sélective l'ensemble de base audit sous-vêtement.

4. Ensemble d'article absorbant à fixation rapide selon la revendication 3, dans lequel l'adhésif de fixation au vêtement est recouvert d'un papier intercalaire amovible.

5. Ensemble d'article absorbant à fixation rapide selon l'une quelconque des revendications précédentes, dans lequel ledit article absorbant comprend une couche de fixation.

6. Ensemble d'article absorbant à fixation rapide selon la revendication 5, dans lequel ladite couche de fixation comprend une pluralité de boucles, lesdites boucles étant structurées et agencées de manière à être engagées et retenues par lesdites structures à crochets.

7. Ensemble d'article absorbant à fixation rapide selon l'une quelconque des revendications précédentes, dans lequel ledit article absorbant comprend un corps perméable au liquide en face de la couche de recouvrement.

8. Ensemble d'article absorbant à fixation rapide selon l'une quelconque des revendications précédentes, dans lequel ledit article absorbant comprend un noyau absorbant qui est disposé entre ladite couche de recouvrement et ladite couche de fixation.
